# EUROPEAN PATENT APPLICATION

(11) **EP 2 324 874 A2**
(43) Date of publication of application: **25.05.2011**
(21) Application number: 09794597.6
(22) Date of filing: 22.06.2009
(51) Int. Cl.: A61M 5/168

(54) **MEDICAL APPARATUS FOR CONTROLLING FLOW**

(30) Priority: 11.07.2008 KR 20080067415
(71) Applicant: Shu, Hyun Bai, Gyeonggi-do 420-720 (KR)
(72) Inventor: Shu, Hyun Bai, Gyeonggi-do 420-720 (KR)
(74) Representative: Stanley, David William
(86) International application number: PCT/KR2009/003344
(87) International publication number: WO 2010/005191

(57) **Abstract**

A medical apparatus for controlling a flow includes: an internal pipe member which has a pipe shape and uniform inner and outer diameters; an external pipe member which has an inner diameter which is more uniform and larger than the outer diameter of the internal pipe member; and a housing means which fixes and supports the internal and external pipe members so that a part of the internal pipe member is inserted into and overlaps with the external pipe member to maintain a uniform gap from an inner wall of the external pipe member in the overlapping part between the internal and external pipe members and which forms a flow path through which a liquid, such as a medicine or the like, supplied from the outside flows into the internal pipe member, moves to a surrounding part of the external pipe member through the uniform gap, and flows out to the outside or flows inversely, wherein the liquid flows at a flow velocity determined according to a length of the overlapping part between the internal and external pipe members and the uniform gap.

## Description

### TECHNICAL FIELD

The present invention relates to a medical apparatus for controlling flow, and more particularly, to a medical apparatus for controlling flow to accurately regulate the dosage of drugs, such as various kinds of injections which are administered to a human body.

### BACKGROUND ART

Various kinds of injections, such as a Ringer's solution, etc., must be continuously administered to a patient at a fixed amount for a fixed time according to an accurate treatment and prescription of a medical specialist before and after an operation or under medical treatment. Although there is a difference between unimportance and importance, if a dosage of a medicine per unit time is small, the medicine is lowly effective. If the dosage of the medicine is excessive, the medicine causes an allergy or even a shock.

A medicine, which is injected into a blood vessel through an injection needle, is generally stored in a Ringer bottle to be continuously supplied from a higher position than a patient by gravity or a contraction pressure of a pressure bag. A pressure from gravity or a contraction pressure of the supplied medicine gradually decreases according to a residual amount of the medicine. If the medicine is directly administered to a human body, the amount of administered medicine is not uniformly maintained. Therefore, it is necessary to control a flow of the medicine supplied from the Ringer bottle or the pressure bag in order to administer a fixed amount of the medicine to the human body.

Examples of conventional simplest structures for controlling a flow of injection medicine include a metal piece clamp and a pinch roller type clamp which are formed of an inelastic material. These clamps pressure a part of a long thin soft hose, which connects a Ringer bottle or a pressure bag storing a medicine to an injection needle, to control an aperture of the hose. Since these clamps control a flow of a medicine by measuring with the eyes, they have very low reliabilities and thus are not desirable to administer an injection medicine requiring precise control.

Conventional devices using capillaries have been suggested to administer medicine requiring precise control. Capillaries increase a frictional resistance of a liquid per unit volume to uniformly maintain a flow velocity of the liquid with respect to an external supply pressure and thus are useful to control a small amount of flow. The flow is determined by attachments, apertures, and lengths of both ends of a capillary but is determined by the apertures and lengths of both ends of a capillary if a supply pressure is sufficient.

Korean Patent Publication No. 10-2005-0099952 suggests a structure which inserts capillaries into a plurality of split soft pipes to uniformly control inflow amounts of a medicine into the pipes and selectively pressures and blocks the pipes to control flow of the medicine.

Korean Patent Publication Nos. 10-2006-0055896 and 10-0075272 and U.S. Patent Nos. 3,785,378 and 3,877,428 suggest dial control type structures which include groove-like capillaries formed in rotors. The grooves are disposed to different depths in radial forms or circular forms, and the radial grooves are selected or effective lengths of the circular grooves vary so as to control a flow.

### DETAILED DESCRIPTION OF THE INVENTION

### TECHNICAL PROBLEM

In a structure using capillaries, elements, including the capillaries (grooves) of conventional apparatuses, are structurally realized as synthetic resin injection molds. The capillaries realized as the synthetic resin injection molds are easily constructed and deformed according to an external temperature, and thus their apertures are changed. In terms of the characteristics of the capillaries, the minute changes in the apertures seriously increase a deviation of a flow control. In other words, if an injection medicine is administered using a conventional apparatus, an administration amount of the medicine is changed according to a room temperature and inner and outer movements of a patient, thereby greatly lowering the reliability of the conventional apparatus.

Inflow amounts into the capillaries are determined according to attachments, apertures, and lengths of ends of the capillaries. It is necessary to maintain the flow amount with an accurate design value since the flow amount is sensitive to changes in factors. However, in a general injection mold structure, it is difficult to maintain a design value, and an error occurs in the design value even in a molding process. Therefore, it is difficult for the general injection mold structure to be provided as a structure capable of precisely controlling linearly a flow amount. However, an injection amount of a medicine requires a widespread linear control according to ingredients of the medicine or states of a patient. Accordingly, the conventional apparatuses have low utility.

The present invention provides a medical apparatus for controlling a flow which is hardly affected by temperature, is designed at a very high degree, maintains its design value, has a high reliability in terms of a flow control, and stably controls the flow from "0" to a maximum range.

### TECHNICAL SOLUTION

According to an aspect of the present invention, there is provided a medical apparatus for controlling a flow, including: an internal pipe member which has a pipe shape and uniform inner and outer diameters; an external pipe member which has an inner diameter which is more uniform and larger than the outer diameter of the internal pipe member; and a housing means which fixes and supports the internal and external pipe members so that a part of the internal pipe member is inserted into and overlaps with the external pipe member to maintain a uniform gap from an inner wall of the external pipe member in the overlapping part between the internal and external pipe members and which forms a flow path through which a liquid, such as a medicine or the like, supplied from the outside flows into the internal pipe member, moves to a surrounding part of the external pipe member through the uniform gap, and flows out to the outside or flows inversely, wherein the liquid flows at a flow velocity determined according to a length of the overlapping part between the internal and external pipe members and the uniform gap.

According to another aspect of the present invention, there is provided a medical apparatus for controlling a flow, including: an internal pipe member which has a pipe shape and uniform inner and outer diameters; an external pipe member which has an inner diameter which is more uniform and larger than the outer diameter of the internal pipe member; a housing means which fixes and supports the internal and external pipe members so that a part of the internal pipe member is inserted into and overlaps with the external pipe member to maintain a uniform gap from an inner wall of the external pipe member in the overlapping part between the internal and external pipe members and which forms a flow path through which a liquid, such as a medicine or the like, supplied from the outside flows into the internal pipe member, moves to a surrounding part of the external pipe member through the uniform gap, and flows out to the outside or flows inversely; and a control means which relatively moves the internal and external pipe members in a pipe-based direction to vary a length of the overlapping part between the internal and external pipe members, wherein the liquid flows at a flow velocity determined according to a length of the overlapping part between the internal and external pipe members and the uniform gap, and the flow velocity varies through the control means.

According to another aspect of the present invention, there is provided a medical apparatus for controlling a flow, including: a pipe member which has a pipe shape and uniform inner and outer diameters; a bar member which has a diameter which is more uniform and smaller than the inner diameter of the pipe member; and a housing means which fixes and supports the pipe member and the bar member so that a part of the pipe member is inserted into and overlaps with the bar member to maintain a uniform gap between an inner wall of the pipe member and an outer surface of the bar member in the overlapping part between the pipe member and the bar member and which forms a flow path through which a liquid, such as a medicine or the like, supplied from the outside flows into an end of the pipe member and then into an other end of the pipe member through the uniform gap and flows out to the outside or flows inversely, wherein the liquid flows at a flow velocity determined according to a length of the overlapping part between the pipe member and the bar member and the uniform gap.

According to another aspect of the present invention, there is provided a medical apparatus for controlling a flow, including: a pipe member which has a pipe shape and uniform inner and outer diameters; a bar member which has a diameter which is more uniform and smaller than the inner diameter of the pipe member; and a housing means which fixes and supports the pipe member and the bar member so that a part of the pipe member is inserted into and overlaps with the bar member to maintain a uniform gap between an inner wall of the pipe member and an outer surface of the bar member in the overlapping part between the pipe member and the bar member and which forms a flow path through which a liquid, such as a medicine or the like, supplied from the outside flows into an end of the pipe member and then into an other end of the pipe member through the uniform gap and flows out to the outside or flows inversely; and a control means which relatively moves the pipe member and the bar member in a pipe-based direction to vary the length of the overlapping part between the pipe member and the bar member, wherein the liquid flows at a flow velocity determined according to the length of the overlapping part between the pipe member and the bar member and the uniform gap, and the flow velocity varies through the control means.

### ADVANTAGEOUS EFFECTS

A medical apparatus for controlling a flow according to the present invention may use metal pipes as internal and external members which structurally function as capillaries. The metal pipes may be more highly precisely managed than synthetic resin molds and may be hardly deformed by changes in an external temperature, thereby greatly improving their reliabilities.

In particular, the internal and external members of the medical apparatus overlap to control a flow amount using an overlapped length of the internal and external members and a tolerance (a gap) between the internal and external members, thereby further minutely controlling the flow amount. In other words, the medical apparatus may accurately administer a medicine to a patient according to the state of the patient or gradients of the medicine to contribute to an increase in a treatment effect.

### DESCRIPTION OF THE DRAWINGS

FIGS. 1A and 1B schematically illustrate general gravity injection type Ringer injection devices to which the present invention is applied;
FIG. 2 is a cross-sectional view of a tube insertion type medical apparatus which controls a flow, according to an embodiment of the present invention;
FIGS. 3A and 3B are left and right side views of the tube insertion type medical apparatus of FIG. 2 seen in an axial direction;
FIG. 4 is a cross-sectional view of a tube insertion type medical apparatus which controls a flow, according to another embodiment of the present invention;
FIG. 5 is a cross-sectional view of a single variable type medical apparatus which controls a flow, according to another embodiment of the present invention;
FIGS. 6A and 6B are left and right side views of the single variable type medical apparatus of FIG. 5 seen in an axial direction;
FIG. 7 is a front view illustrating an outer appearance of the single variable type medical apparatus of FIG. 5;
FIG. 8 is a cross-sectional view of a single variable type medical apparatus which controls a flow, according to another embodiment of the present invention;
FIGS. 9A, 9B, and 9C are cross-sectional views of a single mass storage variable type medical apparatus which controls a flow, according to another embodiment of the present invention; and
FIG. 10 is a cross-sectional view of a single mass storage variable type medical apparatus which controls a flow, according to another embodiment of the present invention.

### BEST MODE

Exemplary embodiments of the present invention will now be described with reference to the attached drawings.

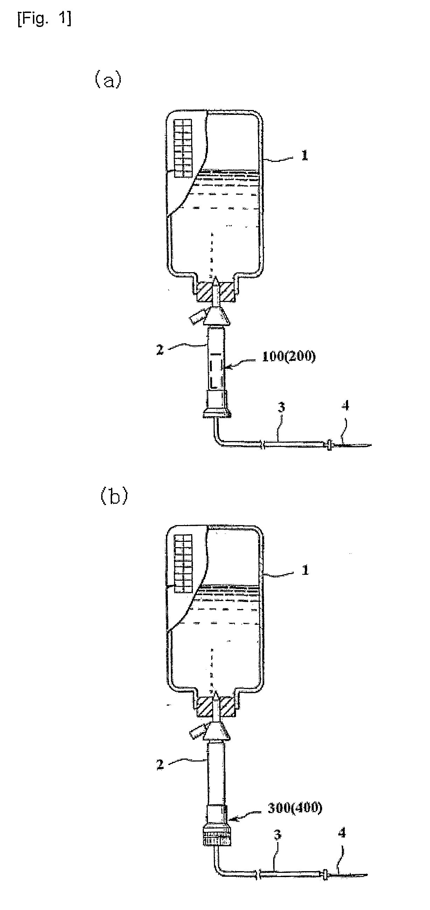

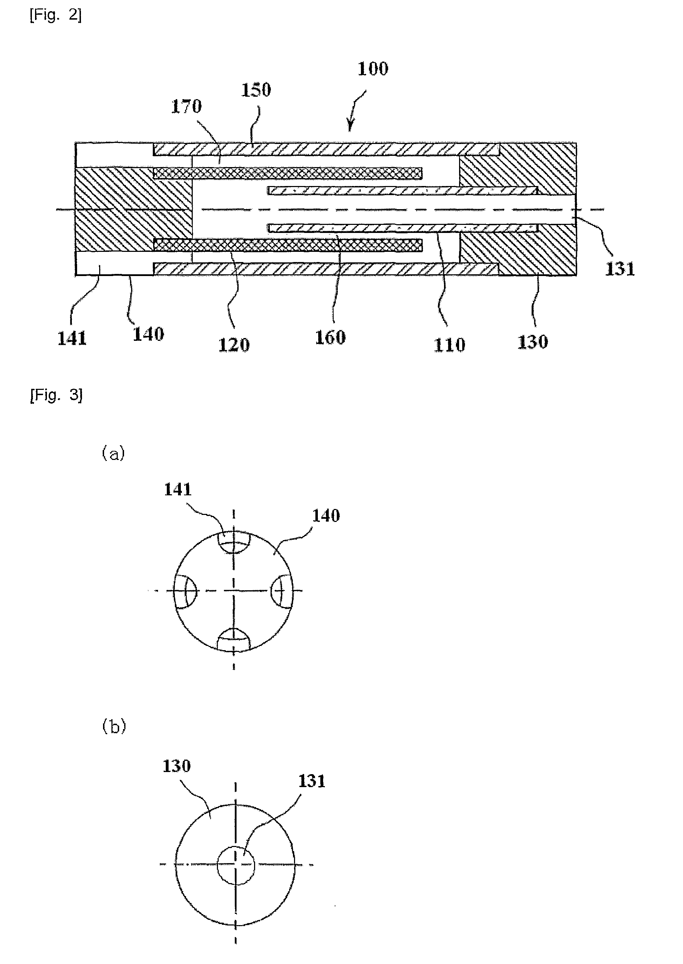

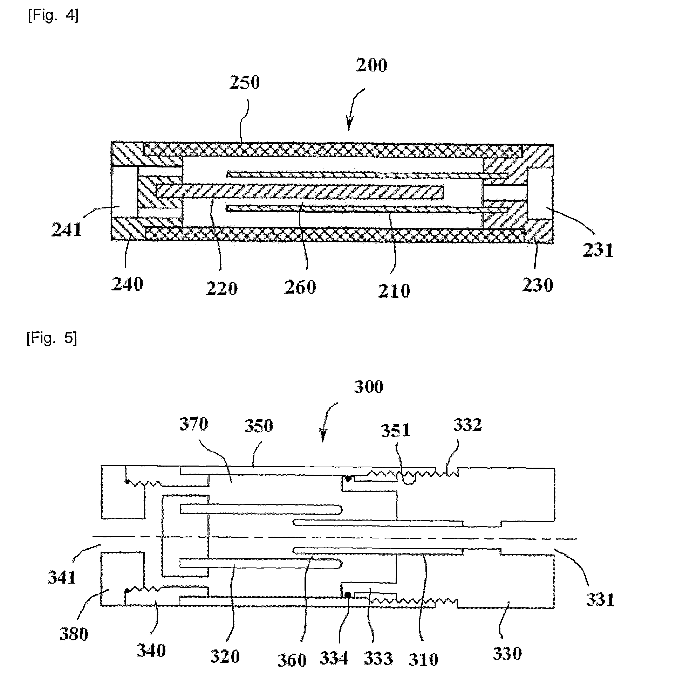

FIGS. 1A and 1B schematically each illustrate a general gravity injection type Ringer injection device. Reference numeral 1 denotes a Ringer bottle which stores a medicine, reference numeral 2 denotes a Ringer spoke tube, reference numeral 3 denotes an injector tube, and reference numeral 4 denotes an injector needle. In the general gravity injection type Ringer injection device, a medical apparatus for controlling a flow, according to the present invention, may be realized as a tube insertion type medical apparatus 100 or 200 which is inserted into the Ringer spoke tube 2 as shown in FIG. 1A or as a single variable type medical apparatus 300 or 400 which is connected between the Ringer spoke tube 2 and the injector tube 4 as shown in FIG. 1B. The tube insertion type medical apparatus 100 or 200 fixes a flow amount according to a design value, e.g., may select a flow amount to 0.5 cc, 1 cc, 2 cc, and 4 cc per unit time according to a patient. Exemplary embodiments of the tube insertion type medical apparatus 100 or 200 are as shown in FIGS. 2 through 4. The single variable type medical apparatus 300 or 400 may arbitrarily control a flow amount per unit time, e.g., may control and administer a desired flow amount within a range between 0 cc and 4 cc or within a narrower or wider range. Exemplary embodiments of the single variable type medical apparatus 300 or 400 and a single mass storage variable type medical apparatus 500 or 600 are as shown in FIGS. 5 through 10.

The tube insertion type medical apparatus 100 shown in FIGS. 2, 3A, and 3B is an exemplary embodiment of a tube insertion type medical apparatus and includes internal and external pipe members 110 and 1120 having pipe shapes, internal and external pipe support members 130 and 140 as a housing means, and a housing member 150. The internal and external pipe members 110 and 120 have pipe shapes with uniform inner and outer diameters and may be provided as synthetic resin pipes, metal pipes, or glass pipes having high corrosion resistance, high thermal resistance, and high strength. The internal and external pipe support members 130 and 140 respectively fix and support the internal and external pipe members 110 and 120 and may be provided as metal molds or heat-resisting resin molds which are respectively processed with the internal and external pipe members 120 and 130 into single bodies.

The housing member 150 may be provided as a transparent glass pipe or a high heat-resisting synthetic resin mold. Combined parts of the members have leakage protection structures so that a medicine does not leak through the combined parts.

The internal and external pipe members 110 and 120 have circular or angulated pipes with uniform inner and outer diameters. The inner diameter of the external pipe member 120 is slightly larger than the outer diameter of the internal pipe member 110. Therefore, when ends of the internal and external pipe members 110 and 120 are respectively fixed to and supported by the internal and external pipe support members 130 and 140, a part of the internal pipe member 110 is inserted into the external pipe member 120, and thus the internal and external pipe members 110 and 120 overlap with each other. Thus, the internal and external pipe members 110 and 120 maintain a uniform gap 160 at the overlapping part. The uniform gap 160 performs a flow control function as a capillary, as described above.

The housing member 150 houses the internal and external pipe members 110 and 120, which overlap with each other, to form a flow path 170 around the internal and external pipe members 110 and 120. The flow path 170 includes input and output ports 131 and 141 which are respectively formed at the internal and external pipe support members 130 and 140 so as to lead to the outside. The input port 131 formed at the internal pipe support member 130 leads to an inner cavity of the internal pipe member 110 supported by the internal pipe support member 130. The output port 141 formed at the external pipe support member 140 leads to the flow path 170 formed around an outer wall of the external pipe member 120, and although one output port 141 is shown, a plurality of output ports 141 may be formed. Here, the input port 131 having a relatively small diameter compared to that of the output port 141, and the output port 141 having a relatively large diameter compared to that of the input port 131 may be an outflow side, but the inflow and outflow sides may be changed with each other.

In the present embodiment, a medicine, which is supplied from the Ringer bottle 1 of FIG. 1A through the Ringer spoke tube 2, flows into the internal pipe member 110 through the input port 131. The medicine moves to the flow path 170 formed around the external pipe member 120 through the uniform gap 160 formed at the overlapping part between the internal and external pipe members 110 and 120. The medicine flows out from the flow path 170 to the injector tube 3 of FIG. 1A through the output port 141. Here, the medicine moves along the internal pipe member 110 up to an end part of the internal pipe member 110 without a great resistance and is controlled at the overlapping part between the internal and external pipe members 110 and 120 at a flow velocity which is determined according to the uniform gap 160 and a length of the overlapping part, thereby uniformly maintaining a minute flow amount of the medicine. An end of the internal pipe member 110 located in the flow path 170 may be processed into a round shape to prevent damage to an inner wall of the external pipe member 120 and reduce a resistance to a fluid in an initial assembly.

The tube insertion type medical apparatus 200 shown in FIG. 4 is another exemplary embodiment of a tube insertion type medical apparatus and includes a pipe member 210, a bar member 220, a pipe support member 230, a bar support member 240, a housing member 250. In the present embodiment, except for the bar member 220, the other members are substantially equal to corresponding elements of the embodiment of FIG.2.

The bar member 220 has a smaller diameter than an inner diameter of the pipe member 210, and a part of the bar member 220 is inserted into and overlaps with the pipe member 210. Therefore, the bar member 220 maintains a uniform gap 260, which functions as a capillary, from an inner wall of the pipe member 210, in the overlapping part. A medicine which flows into the pipe member 210 through a port 231 flows at a limited velocity through the uniform gap 260 and flows from an end of the pipe member 210 to the outside through a port 241 without a great resistance. A fixed flow amount of the medicine which is designed according to the uniform gap 260 in the overlapping part between the pipe member 210 and the bar member 220 and a length of the overlapping part may be maintained.

The single variable type medical apparatus 300 shown in FIGS. 5, 6A, 6B, and 7 is an exemplary embodiment of a single variable type medical apparatus and includes internal and external pipe members 310 and 320 having pipe shapes, internal and external pipe support members 330 and 340 as a housing means, and a housing member 350. Screw members 332 and 351 are respectively formed as control means at the internal pipe support member 330 and the housing member 350 to be combined in male and female forms with each other. A ring-shaped protrusion 333 is formed as a leak prevention structure at the internal pipe support member 310 and extends toward an inner surface of the housing member 350. A packing ring 334 s interposed between an end of the ring-shaped protrusion 333 and the inner surface of the housing member 350 and is formed of an elastic adhesive material so as to slide and maintain its adhesiveness. A nipple 380 is combined with the external pipe support member 340 to be connected to the Ringer spoke tube 2 of FIG. 1B. The injector tube 3 of FIG. 1B is connected to a port 331 of the internal pipe support member 310. In other words, in the single variable type medical apparatus 300 of the present embodiment, the port 331 is an inflow side of an inflow path of a medicine, the 331 is an outflow side, and a flow path of the medicine is in an opposite direction to that described in the embodiment of FIG. 2.

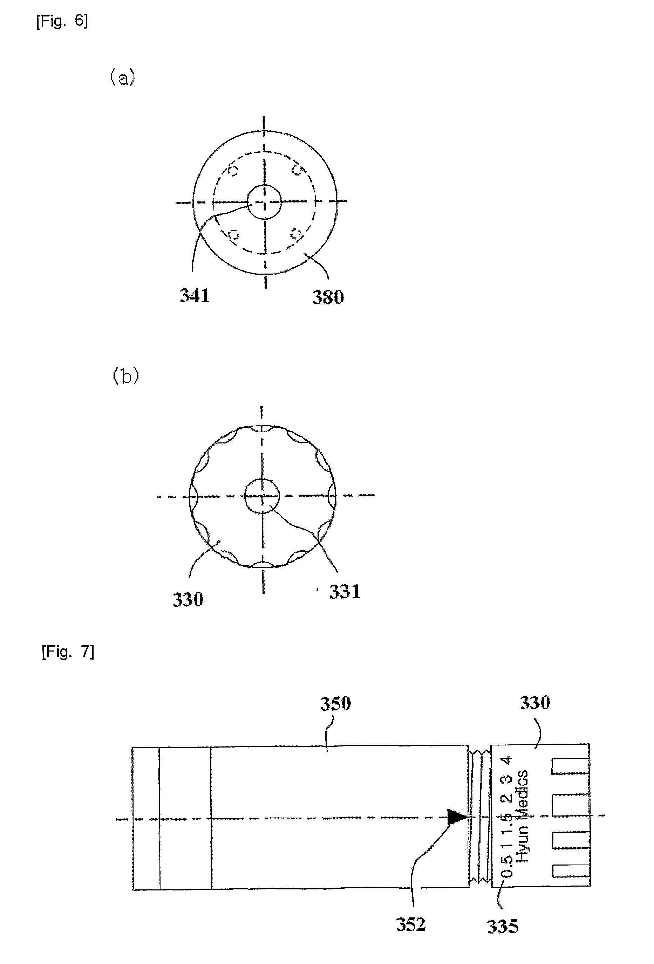

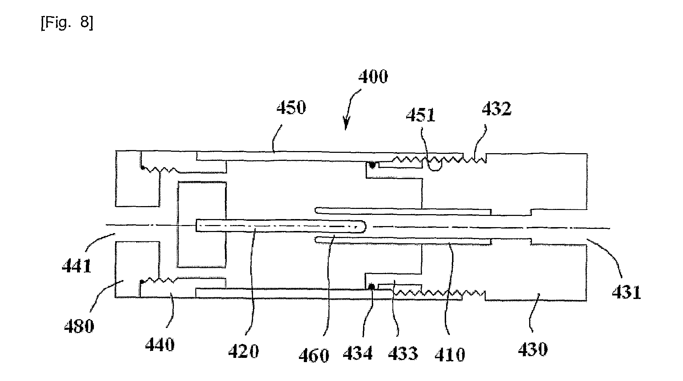

In the single variable type medical apparatus 300 according to the present embodiment, the internal pipe support member 330 and the housing member 350 are screwed to each other and thus are mutually elastically controlled in a pipe-based direction, which is a screwing direction of the internal pipe support member 330 and the housing member 350. The length of the overlapping part between the internal and external pipe members 310 and 320 is elastically controlled according to the elastic control. In this case, a fluid velocity varies at the overlapping part between the internal and external pipe members 310 and 320 according to the degree of elastic control of the overlapping part, thereby increasing or decreasing a flow amount of the medicine passing per unit time. Therefore, a minute flow amount of the medicine may be accurately controlled according to the states of a patient or the administered medicine to uniformly administer the medicine. Referring to FIG. 7, flow gradations 335 are printed on an outer surface of the internal pipe member 310, and a flow indicator 352 is printed on an outer surface of the housing member 350. Therefore, a desired flow amount may be easily selectively controlled through the flow gradations 335 and the flow indicator 352.
The single variable type medical apparatus medical apparatus 400 shown in FIG. 8 includes a pipe member 410, a bar member 420, a pipe support member 430, a bar support member 440, and a housing member 450. In the present embodiment, the bar member 420 is substantially equal to the bar member 220 of FIG. 4, and the other members are substantially equal to those of the embodiment of FIG. 5. A length of an overlapping part between the pipe member 410 and the bar member 420 may be controlled by an elastic control between the pipe support member 430 and the housing member 450 to control a desired flow amount.

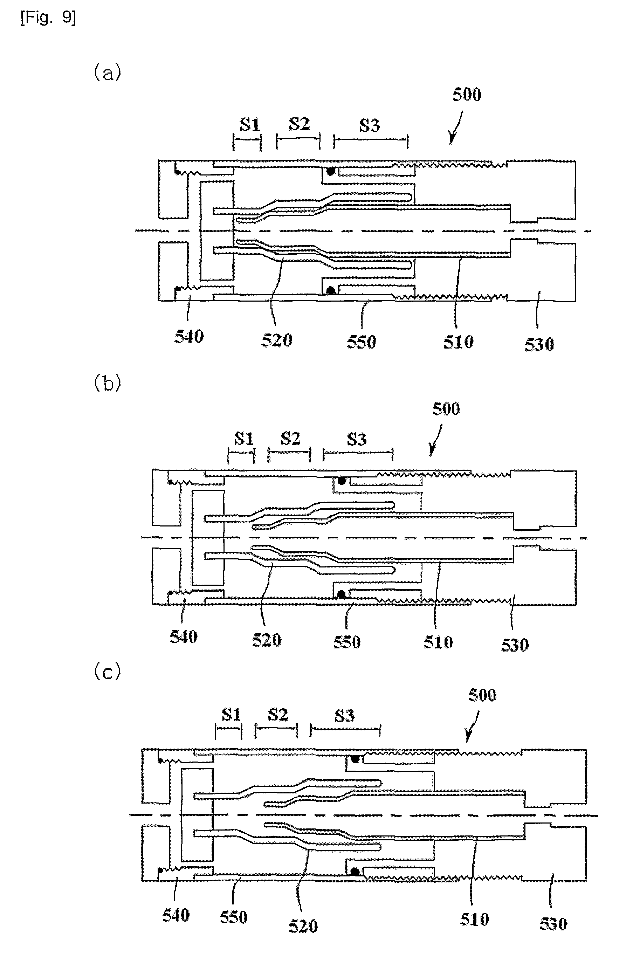

The single mass storage variable type medical apparatus 500 for controlling a flow, shown in FIGS. 9A through 9C, is characterized in that a flow amount may be controlled to a further expanded range. For example, if a large amount of an injection, such as a blood transfusion, is required within a short time during an operation, and the injection is to be re-controlled to a minute amount according to progress of the operation, the single mass storage variable type medical apparatus 500 of the present embodiment may be useful. In the present embodiment, internal and external pipe members 510 and 520 are formed in stages, and other members are substantially equal to those of the single variable type medical apparatuses which have been described above. The internal pipe member 510 has inner and outer diameters which decrease in stages toward an end thereof and are uniform in each of the stages. The external pipe member 520 has a corresponding shape to that of the internal pipe member 510 and an inner diameter which increases in stages toward an end thereof. The inner diameter of the external pipe member 520 is uniform in each of the stages and is slightly larger than the external diameter of the internal pipe member 510 in each of the stages. An external diameter of the external pipe member 520 is shown as having the same form as the internal diameter of the external pipe member 520 but may be uniform as a whole. Here, if each of the internal and external pipe members 510 and 520 is divided into first, second, and third stage control sections S1, S2, and S3 from a part having a small diameter to a part having a large diameter, in each stage, a maximum overlapping length in the first, second, and third stage control sections S1, S2, and S3 must be times longer than a maximum overlapping length in the first stage control sections S1. In other words, a maximum overlapping length in the second stage control sections S2 is set to two times or more the maximum overlapping length in the first stage control sections S1, and a maximum overlapping length in the third stage control sections S3 is set to three times or more the maximum overlapping length between the first stage control sections S1.

FIG. 9A is a cross-sectional view illustrating a first stage control state. In this state, an overlapping part in the first stage control sections S1 of the internal and external pipe members 510 and 520 having the smallest diameters performs a minute control function to control a flow amount to a minute amount.

In a second stage control state of FIG. 9B, an end part of the internal pipe member 510 is outside of the first stage control section S1 of the external pipe member 520 to start a flow amount control. In this state, an overlapping part in the second stage control sections S2, which are second stage diameter parts of the internal and external pipe members 510 and 520, performs the minute control function to control the flow amount to an intermediate range.

In a third stage control state of FIG. 9C, the end part of the internal pipe member 510 is outside of the second stage control section S2 of the external pipe member 520 to start the flow amount control. In this state, an overlapping part in the third stage control sections S2, which are third stage diameter parts of the internal and external pipe members 510 and 520, performs the minute control function to control the flow amount to a maximum range.

In the present embodiment, the three stages are shown in FIGS. 9A through 9C, but two or more stages may be embodied.

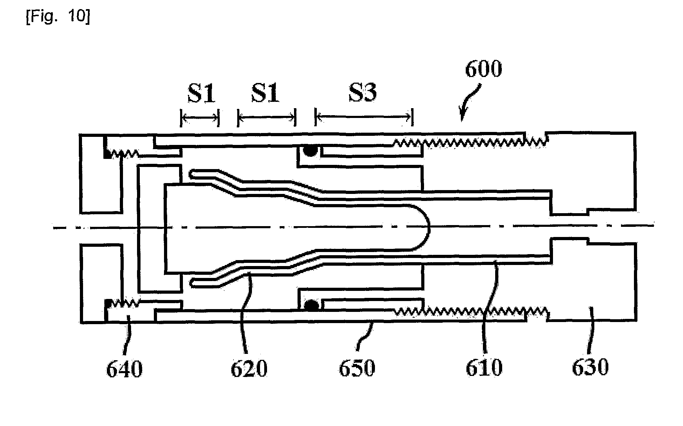

The single mass storage variable type medical apparatus 600 for controlling a flow, shown in FIG. 10, includes a multi-stage pipe member 610, a multi-stage bar member 620, a pipe support member 630, a bar support member 640, and a housing member 650. The multi-stage pipe member 610 and the multi-stage bar member 620 respectively correspond to the internal and external pipe members 510 and 520 of the embodiment of FIGS. 9A through 9C, and the other members are substantially equal to those of FIG. 9A through 9C.

Like the previous embodiment, the present embodiment enables a minute control function in each stage and enables a flow control to a flow range expanded in each stage.

The above-described embodiments exemplify a tube insertion type medical apparatus which fixes a flow amount, and a single variable type and a single mass storage variable type medical apparatus which can control a flow amount. However, various types including a single fixing type fixing a flow amount may be embodied.

## Claims

1. A medical apparatus for controlling a flow, comprising:
an internal pipe member which has a pipe shape and uniform inner and outer diameters;
an external pipe member which has an inner diameter which is more uniform and larger than the outer diameter of the internal pipe member; and
a housing means which fixes and supports the internal and external pipe members so that a part of the internal pipe member is inserted into and overlaps with the external pipe member to maintain a uniform gap from an inner wall of the external pipe member in the overlapping part between the internal and external pipe members and which forms a flow path through which a liquid, such as a medicine or the like, supplied from the outside flows into the internal pipe member, moves to a surrounding part of the external pipe member through the uniform gap, and flows out to the outside or flows inversely,
wherein the liquid flows at a flow velocity determined according to a length of the overlapping part between the internal and external pipe members and the uniform gap.

2. The medical apparatus of claim 1, wherein the housing means comprises:
an internal pipe support member which supports an end of the internal pipe member and comprises a port which leads into the end of the internal pipe member;
an external pipe support member which supports an end of the external pipe member and comprises a port which leads into an inside of the end of the external pipe member and leads into an outer surrounding part; and
a housing member which is connected between the internal and external pipe support members at a gap for the flow path from the external pipe member.

3. A medical apparatus for controlling a flow, comprising:
an internal pipe member which has a pipe shape and uniform inner and outer diameters;
an external pipe member which has an inner diameter which is more uniform and larger than the outer diameter of the internal pipe member;
a housing means which fixes and supports the internal and external pipe members so that a part of the internal pipe member is inserted into and overlaps with the external pipe member to maintain a uniform gap from an inner wall of the external pipe member in the overlapping part between the internal and external pipe members and which forms a flow path through which a liquid, such as a medicine or the like, supplied from the outside flows into the internal pipe member, moves to a surrounding part of the external pipe member through the uniform gap, and flows out to the outside or flows inversely; and
a control means which relatively moves the internal and external pipe members in a pipe-based direction to vary a length of the overlapping part between the internal and external pipe members,
wherein the liquid flows at a flow velocity determined according to a length of the overlapping part between the internal and external pipe members and the uniform gap, and the flow velocity varies through the control means.

4. The medical apparatus of claim 3, wherein the housing means comprises:
an internal pipe support member which supports an end of the internal pipe member and comprises a port which leads into the end of the internal pipe member;
an external pipe support member which supports an end of the external pipe member and comprises a port which leads into an inside of the end of the external pipe member and leads into an outer surrounding part; and
a housing member which is connected between the internal and external pipe support members at a gap for the flow path from the external pipe member,
wherein the control means is constituted by screwing the internal pipe support member and the housing member of the housing means to each other so that the internal pipe support member and the housing member are mutually elastically controlled in the pipe-based direction.

5. The medical apparatus of claim 3 or 4, wherein the internal and external pipe members are constituted in stages in which diameters of the internal and external pipe 14 members vary by stages so that a flow amount is controlled within each range according to a length and a gap between an overlapping part of the internal and external pipe members, and the range of the flow amount is controlled in each of the stages.

6. A medical apparatus for controlling a flow, comprising:
a pipe member which has a pipe shape and uniform inner and outer diameters;
a bar member which has a diameter which is more uniform and smaller than the inner diameter of the pipe member; and
a housing means which fixes and supports the pipe member and the bar member so that a part of the pipe member is inserted into and overlaps with the bar member to maintain a uniform gap between an inner wall of the pipe member and an outer surface of the bar member in the overlapping part between the pipe member and the bar member and which forms a flow path through which a liquid, such as a medicine or the like, supplied from the outside flows into an end of the pipe member and then into an other end of the pipe member through the uniform gap and flows out to the outside or flows inversely,
wherein the liquid flows at a flow velocity determined according to a length of the overlapping part between the pipe member and the bar member and the uniform gap.

7. A medical apparatus for controlling a flow, comprising:
a pipe member which has a pipe shape and uniform inner and outer diameters;
a bar member which has a diameter which is more uniform and smaller than the inner diameter of the pipe member; and
a housing means which fixes and supports the pipe member and the bar member so that a part of the pipe member is inserted into and overlaps with the bar member to maintain a uniform gap between an inner wall of the pipe member and an outer surface of the bar member in the overlapping part between the pipe member and the bar member and which forms a flow path through which a liquid, such as a medicine or the like, supplied from the outside flows into an end of the pipe member and then into an other end of the pipe member through the uniform gap and flows out to the outside or flows inversely; and
a control means which relatively moves the pipe member and the bar member in a pipe-based direction to vary the length of the overlapping part between the pipe member and the bar member,
wherein the liquid flows at a flow velocity determined according to the length of the overlapping part between the pipe member and the bar member and the uniform gap, and the flow velocity varies through the control means.
